(19) **Europäisches Patentamt**
**European Patent Office**
**Office européen des brevets**

(11) **EP 1 704 273 B1**

(12) **EUROPEAN PATENT SPECIFICATION**

(45) Date of publication and mention
of the grant of the patent:
**02.09.2009 Bulletin 2009/36**

(51) Int Cl.:
***D04H 13/00*** (2006.01)   ***B32B 5/26*** (2006.01)

(21) Application number: **04811156.1**

(86) International application number:
**PCT/US2004/038340**

(22) Date of filing: **15.11.2004**

(87) International publication number:
**WO 2005/066405 (21.07.2005 Gazette 2005/29)**

(54) **NONWOVEN WEBS HAVING REDUCED LINT AND SLOUGH**

VLIESSTOFFE MIT WENIGER FUSSELN UND FASERABSCHEIDUNG

BANDES NON TISSES PRESENTANT UN PELUCHAGE ET UN EBOULEMENT REDUITS

(84) Designated Contracting States:
**DE FR GB IT**

(30) Priority: **30.12.2003 US 748454**

(43) Date of publication of application:
**27.09.2006 Bulletin 2006/39**

(73) Proprietor: **KIMBERLY-CLARK WORLDWIDE, INC.
Neenah, WI 54956 (US)**

(72) Inventors:
• **CLOSE, Kenneth, B.
New London, WI 54961 (US)**
• **ANDERSON, Gary, V.
Larsen, WI 54947 (US)**
• **BAER, David, J.
Oshkosh, WI 54901 (US)**
• **KOPACZ, Thomas, J.
Omro, WI 54963 (US)**

• **LINDSAY, Jeffrey, D.
Appleton, WI 54915 (US)**
• **CHEN, Fung-jou
Appleton, WI 54915 (US)**
• **BEDNARZ, Julie Marie
Neenah, WI 54956 (US)**

(74) Representative: **Davies, Christopher Robert et al
Frank B. Dehn & Co.
St Bride's House
10 Salisbury Square
London
EC4Y 8JD (GB)**

(56) References cited:
| | |
|---|---|
| EP-A- 0 205 242 | EP-A- 0 590 307 |
| WO-A-01/00917 | WO-A-90/04060 |
| WO-A-92/16364 | WO-A-03/048453 |
| WO-A-20/04061228 | US-A- 4 436 780 |

**EP 1 704 273 B1**

**Description**

## BACKGROUND OF THE INVENTION

Pulp fibers, such as softwood fibers and hardwood fibers, are incorporated into numerous nonwoven materials. The nonwoven materials, in tum, are used in almost a limitless variety of applications. For instance, pulp fibers are used to form tissue products, including facial tissues, bath tissues, paper towels, industrial wipers, and the like. Pulp fibers are also incorporated into composite nonwoven materials that may contain pulp fibers in combination with polymeric fibers. Composite nonwoven materials may be used, for instance, to make wet wipes, tablecloths, surgical drapewear, bandages, and absorbent structures for incorporation into disposable absorbent garments such as diapers, feminine care products, and adult incontinence products.

Pulp fibers may be engineered to have great absorbency properties and can feel soft to the skin when incorporated into the above nonwoven materials. Further, pulp fibers are relatively inexpensive to obtain, which permits the production of relatively inexpensive products that may be disposed of after a single use.

Nonwoven materials incorporating pulp fibers are designed to include several important properties. For example, in some applications, the nonwoven materials should have good bulk, a soft feel, and should have good strength. Unfortunately, however, when steps are taken to increase one property of the material, other characteristics of the material are often adversely affected.

WO 90/04 060 and EP-A-0 205 242 disclose nonwoven wiper laminate materials.

For instance, in many applications, pulp fibers are treated with chemical debonders which are designed to reduce fiber bonding between the pulp fibers. Reducing fiber bonding can increase the softness of the material. Chemical debonders, however, can also sometimes adversely affect the strength of the nonwoven material, especially when the material comprises a tissue product.

For instance, the inclusion of chemical debonders into nonwoven materials can result in loosely bound fibers that extend from the surface of the nonwoven material. During use, when the nonwoven materials are subjected to shear forces, the loosely bound fibers can become liberated from the material and can remain suspended in the air or can result in slough, which is when bundles or pills of fibers become transferred onto an adjacent surface, such as the skin or clothes of the user.

Slough and lint can be particularly problematic in creped tissue, where the surface disruption caused by creping can result in liberated fibers that may be released from the sheet as lint during use. Layered tissues, with high hardwood content in an outer layer, can also be subject to severe linting problems.

Indeed, lint and slough generally remain a problem faced by the manufacturers of wiping products that contain pulp fibers, such as tissue products and pre-saturated wet wipes. Efforts to reduce slough and lint without a noticeable loss of bulk and softness have not been completely successful. Thus, a need currently exists for a method for reducing lint and slough in nonwoven materials containing pulp fibers.

## DEFINITIONS

As used herein, the term "meltblown fibers" means fibers formed by extruding a molten thermoplastic material through a plurality of fine, usually circular, die capillaries as molten threads or filaments into a high velocity gas (e.g. air) stream which attenuates the filaments of molten thermoplastic material to reduce their diameter, which can be to microfiber diameter. Thereafter, the meltblown fibers are carried by the high velocity gas stream and are deposited on a collecting surface to form a web of randomly disbursed meltblown fibers. Such a process is disclosed, for example, in U.S. Patent No. 3,849,241 to Butin.

As used herein, the term "spunbonded fibers" refers to small diameter fibers which are formed by extruding a molten thermoplastic material as filaments from a plurality of fine, usually circular, capillaries of a spinnerette with the diameter of the extruded filaments then being rapidly reduced as by, for example, eductive drawing or other well-known spun-bonding mechanisms. The production of spunbonded nonwoven webs is illustrated in patents such as, for example, in U.S. Patent No. 4,340,563 to Appel, et al., and U.S. Patent No. 3,692,618 to Dorschner, et al.

As used herein, the term "conform" means a nonwoven composite material of air-formed matrix material comprising thermoplastic polymeric meltblown fibers such as, for example, microfibers having an average fiber diameter of less than about 10 microns, and a multiplicity of individualized absorbent fibers such as, for example, wood pulp fibers disposed throughout the matrix of polymer microfibers and engaging at least some of the microfibers to space the microfibers apart from each other. The absorbent fibers are interconnected by and held captive within the matrix of microfibers by mechanical entanglement of the microfibers with the absorbent fibers, the mechanical entanglement and Interconnection of the microfibers and absorbent fibers alone forming a coherent Integrated fibrous structure. These materials are prepared according to the descriptions in U.S. Patent No. 4,100,324 to Anderson, et al., U.S. Patent No. 5,508102 to Georger, et al. (equivalent to EP-A-0 590 307), U.S. Patent No. 5,284,703 to Everhart, et al., U.S. Patent No. 5,350,624 to Georger, et al. (equivalent to D3), and U.S. Patent No. 5,385,775 to Wright.

As used herein, the term "microfibers" means small diameter fibers having an average diameter not greater than about 100 microns, for example, having an average diameter of from about 0.5 microns to about 50 microns, or more particularly, microfibers may have an average diameter of from about 4 microns to about 40 microns.

As used herein, the term "autogenous bonding" means bonding provided by fusion and/or self-adhesion of fibers and/or filaments without an applied external adhesive or bonding agent. Autogenous bonding can be provided by contact between fibers and/or filaments while at least a portion of the fibers and/or filaments are semi-molten or tacky. Autogenous bonding may also be provided by blending a tackifying resin with the thermoplastic polymers used to form the fibers and/or filaments. Fibers and/or filaments formed from such a blend can be adapted to self-bond with or without the application of pressure and/or heat. Solvents may also be used to cause fusion of fibers and filaments which remains after the solvent is removed.

As used herein, the terms "stretch-bonded laminate" or "composite elastic material" refers to a fabric material having at least one layer of nonwoven web with at least one of the layers of nonwoven web being elastic and at least one layer of the nonwoven web being non-elastic, e.g., a gatherable layer. The elastic nonwoven web layer(s) are joined or bonded to at least two locations to the non-elastic nonwoven web layer(s). Preferably, the bonding is at intermittent bonding points or areas while the nonwoven web layer(s) are in juxtaposed configuration and while the elastic nonwoven web layer(s) have a tensioning force applied thereto in order to bring the elastic nonwoven web to a stretched condition. Upon removal of the tensioning force after joining of the web layers, an elastic nonwoven web layer will attempt to recover to its unstretched condition and will thereby gather the non-elastic nonwoven web layer between the points or areas of joining of the two layers. The composite material is elastic in the direction of stretching of the elastic layer during joining of the layers and can be stretched until the gathers of the non-elastic nonwoven web or film layer have been removed. A stretch-bonded laminate may include more than two layers. For example, the elastic nonwoven web or film may have a non-elastic nonwoven web layer joined to both of its sides while it is in a stretched condition so that a three layer nonwoven web composite is formed having the structure of gathered non-elastic (nonwoven web or film)/elastic (nonwoven web or film)/gathered non-elastlc (nonwoven web or film). Yet other combinations of elastic and non-elastic layers can also be utilized. Such composite elastic materials are disclosed, for example, by U.S. Patent No. 4,720,415 to Vander Wielen, et al., and U.S. Patent No. 5,385,775 to Wright.

In accordance with the present invention there is provided a wet wipe as defined in claim 1. In general, the present invention is directed to a process for producing nonwoven materials having reduced lint and slough. The present invention is also directed to the materials produced by the process. The nonwoven materials contain pulp fibers and, in accordance with the present invention, include a meltblown "veneer" applied to at least one side of the material that has been found to greatly reduce lint and slough without substantially affecting the other properties of the material.

Nonwoven materials as described above are used in industrial wipers, and the like. In accordance with the present invention, the nonwoven material is made from a composite fibrous web containing pulp fibers in combination with polymeric fibers. These composite materials may be used in various wiping applications. For instance, the materials may be used to construct pre-saturated wet wipes.

In one particular embodiment, the present invention is directed to a nonwoven web comprising pulp fibers. The nonwoven web has a first side and a second and opposite side. Meltblown fibers are applied to the first side of the web in a manner so as to reduce lint and slough. The meltblown fibers may be, for instance, distributed over the surface of the first side of the nonwoven web. The meltblown fibers have been found to reduce lint and slough when placed on the nonwoven web at extremely low levels, such as less than about 8 gsm. In other embodiments, for instance, the meltblown fibers may be present on the web in an amount less than about 6 gsm, in an amount less than about 4 gsm, in an amount less than about 2 gsm, and even in amounts less than 1 gsm for some applications.

The meltblown fibers applied to the web can have a diameter of less than about 10 microns, such as less than about 5 microns. The fibers may comprise continuous filaments. The meltblown fibers may be made from various polymeric materials, such as styrene-butadiene copolymers, polyvinyl acetate homopolymers, vinyl acetate ethylene copolymers, vinyl acetate acrylic copolymers, ethylene vinyl chloride copolymers, ethylene vinyl chloride-vinyl acetate terpolymers, acrylic polyvinyl chloride polymers, acrylic polymers, nitrile polymers, and waxes such as a paraffin wax. The meltblown fibers may be made from thermosetting polymers, photocuring polymers, and thermoplastic polymers. In one particular embodiment, the meltblown fibers are made from ethylene vinyl alcohol or from an ethylene vinyl acetate copolymer.

In one embodiment, the meltblown fibers comprise a polymer with a plurality of hydrophilic groups such as carboxylic acid groups or salts thereof, or hydroxyl groups, which, in some cases, can help provide good adhesion with cellulose even when the cellulose is wet. Such adhesives can comprise polyvinyl alcohols or EVA (ethylene vinyl acetate), and may include, by way of example, the EVA HYSOL® hotmelts of Henkel Loctite Corporation (Rocky Hill, Connecticut), including 232 EVA HYSOL®; 236 EVA NYSOL®, 1942 EVA HYSOL®, 0420 EVA HYSOL® SPRAYPAC® , 0437 EVA HYSOL® SPRAYPAC®, CoolMelt EVA HYSOL®, QuikPac EVA HYSOL®, SuperPac EVA HYSOL®, and WaxPac EVA HYSOL®. EVA-based adhesives can be modified through the addition of tackifiers and other conditioners, such as Wingtack 86 tackifying resin manufactured by Goodyear Corporation (Akron, Ohio).

In another embodiment, the meltblown fibers comprise an elastomeric component such as block copolymers derived from styrene-butadiene systems, such as styrene-ethylene-butylene-styrene (SEBS), styrene-butadiene-styrene (SBS), styrene-isoprene-styrene (SIS), and the like. Useful block copolymers may also be polyether block copolymers (e.g., PEBAX), copolyester polymers, polyester/polyether block polymers, and the like.

According to the present invention the nonwoven web comprises a coform web containing a mixture of pulp fibers and polymeric fibers. The coform web may contain pulp fibers, for instance, in an amount greater than about 40% by weight, such as from about 50% to about 80% by weight. The polymeric fibers may comprise meltblown fibers made from a polyolefin polymer.

When treating a coform web, the meltblown fibers applied to the web are made from a polymer that is compatible with the polymeric fibers contained within the coform web. For instance, the meltblown fibers can be made from a polyolefin polymer.

Coform webs made according to the present invention are used to produce a wet wipe that is pre-saturated with a wiping solution. The wet wipe comprises a first coform web, a second coform web, and an elastic layer positioned between the first coform web and the second coform web. Each of the coform webs may be treated with meltblown fibers in accordance with the present invention. In particular, the coform webs are treated on the side of the web that forms an exterior surface of the stretch-bonded laminate.

Of particular advantage, it has been discovered that coform webs may be treated with meltblown fibers according to the present invention without significantly adversely affecting the softness properties and wiping properties of the web. For instance, coform webs treated according to the present invention may have a cup crush of less than about 150 g/cm, such as less than about 125 g/cm. The coform webs may also have a density of less than about 0.08 g/cm$^3$, such as less than about 0.07 g/cm$^3$.

Other preferred features of the present invention are discussed in greater detail below.

## BRIEF DESCRIPTION OF THE DRAWINGS

A full and enabling disclosure of the present invention, including the best mode thereof to one skilled in the art, is set forth more particularly in the remainder of the specification, including reference to the accompanying figures, in which:

Figure 1 is a schematic flow diagram of one embodiment of a process for applying meltblown fibers to a coform web in accordance with the present invention;

Figure 2 is a schematic flow diagram of one embodiment of a process for forming stretch-bonded laminates in accordance with the present invention; and

Figure 3 is a perspective view of one embodiment of a process for forming an elastic layer for use in laminates made according to the present invention.

Repeat use of reference characters in the present specification and drawings is intended to represent the same or analogous features or elements of the present invention.

## DETAILED DESCRIPTION OF THE INVENTION

It is to be understood by one of ordinary skill in the art that the present discussion is a description of exemplary embodiments only, and is not intended as limiting the broader aspects of the present invention.

In general, the present invention is directed to a process for reducing lint and slough levels In nonwoven webs containing pulp fibers. According to the present invention, a relatively small amount of a polymeric material is applied to at least one surface of a nonwoven web in order to reduce lint and slough. The polymeric material may be in the form of fibers or droplets. In one particular embodiment of the present invention, for instance, a veneer comprised of meltblown fibers is applied to at least one side of the nonwoven web.

At very low add-on levels, it has been discovered, for instance, that meltblown fibers may be applied to a nonwoven web for reducing lint and slough without adversely affecting many other properties of the material. In fact, in some embodiments, the meltblown fibers are not discernible, and yet can reduce slough levels by greater than 30%.

In general, any nonwoven material containing pulp fibers may be treated according to the teachings of the present invention. For instance, the nonwoven material may be an industrial wiper, and the like. Paper towels and other wiping products have a basis weight of from about 40 gsm to about 80 gsm.

The present invention is particularly well suited to reducing lint and slough levels in composite webs, such as coform webs. In fact, coform webs may be made according to the present invention having reduced lint and slough levels while still having a low cup crush, a low density, and maintaining a desired level of strength and tear resistance.

Further, coform webs made according to the present invention can actually exhibit a surface having a reduced coefficient of friction. Thus, when used as a wiping product, the webs have a greater tendency to slide across an adjacent surface which may be, for instance, a countertop or a user's skin.

Products Containing Coform Webs

The teachings of the present invention are well suited to reducing lint and slough levels in coform webs. In particular, it was discovered that a very light treatment of meltblown fibers to a coform web can reduce lint and slough levels while maintaining the flexibility of the web. In fact, since the meltblown fibers can be applied at such low amounts, the softness of the web is not substantially affected. For instance, coform webs made according to the present invention may have a cup crush of less than about 150 g/cm, such as less than about 125 g/cm. In other embodiments, it is believed that the cup crush of coform webs made according to the present invention can be less than 120 g/cm, or less than about 115 g/cm. In fact, the meltblown fibers have also been found to decrease the coefficient of friction on the treated side of the web allowing the coform web to slide more easily across surfaces, which further reduces lint and further improves the perceive softness of the web.

The density of coform webs made according to the present invention can also be relatively low. For instance, the density may be less than about 0.08 g/cm$^3$, such as less than about 0.07 g/cm$^3$.

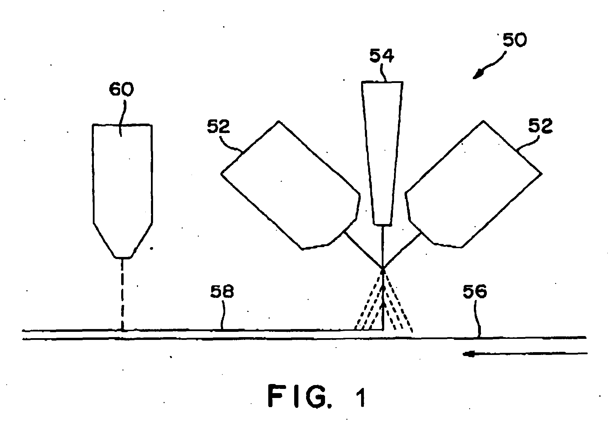

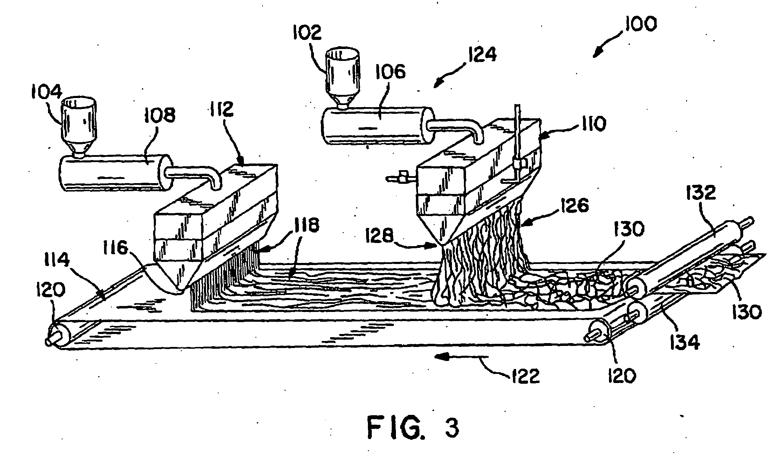

Referring to **Figure 1**, one embodiment of a process for forming coform webs in accordance with the present invention is shown. The coform webs are made from microfibers formed by extrusion processes such as, for example, mettblowing processes or spunbonding processes. In the embodiment illustrated in **Figure 1**, thermoplastic polymer microfibers are formed from extruder banks generally **50** comprising, in this embodiment, meltblowing extruders **52**. The microfibers are blended with individualized wood pulp fibers exiting a pulp generator **54**. Although two meltblowing extruders **52** are shown in **Figure 3,** it should be understood that more or less extruders may be used.

From the extruders **52** and the pulp generator **54,** a coform web **58** is created on a forming surface **56.**

The coherent integrated fibrous structure **58** can be formed by the microfibers and wood pulp fibers without any adhesive, molecular or hydrogen bonds between the two different types of fibers. The wood pulp fibers are preferably distributed uniformly throughout the matrix of microfibers to provide a homogeneous material. The material is formed by initially forming a primary air stream containing the melt blown microfibers, forming a secondary air stream containing the wood pulp fibers, merging the primary and secondary streams under turbulent conditions to form an integrated air stream containing a thorough mixture of the microfibers and wood pulp fibers, and then directing the integrated air stream onto the forming surface **56** to air form the fabric-like material. The microfibers are in a soft nascent condition at an elevated temperature when they are turbulently mixed with the wood pulp fibers in air.

In one embodiment, the coform layer(s) can have from about 20-50 wt. % of polymer fibers and about 80-50 wt. % of pulp fibers. For instance, the ratio of polymer fibers to pulp fibers can be from about 25-40 wt. % of polymer fibers and about 75-60 wt. % of pulp fibers. In another embodiment, the ratio of polymer fibers to pulp fibers can be from about 30-40 wt. % of polymer fibers and about 70-60 wt. % of pulp fibers. For instance, the ratio of polymer fibers to pulp fibers can be about 35 wt. % of polymer fibers and about 65 wt. % of pulp fibers.

Non-limiting examples of the polymers suitable for forming coform webs are polyolefin materials such as, for example, polyethylene, polypropylene and polybutylene, including ethylene copolymers, propylene copolymers and butylene copolymers thereof. A particularly useful polypropylene is Basell PF-105. Additional polymers are disclosed in U.S. Patent No. 5,385,775 to Wright.

Fibers of diverse natural origin are applicable to the invention. Digested cellulose fibers from softwood (derived from coniferous trees), hardwood (derived from deciduous trees) or cotton linters can be utilized. Fibers from Esparto grass, bagasse, kemp, flax, and other lignaceous and cellulose fiber sources may also be utilized as raw material in the invention. For reasons of cost, ease of manufacture and disposability, in one embodiment, the fibers are those derived from wood pulp (i.e., cellulose fibers). A commercial example of such a wood pulp material is available from Weyerhaeuser as CF-405. Other commercially available wood pulp materials include Georgia Pacific Golden Isles Fluff Pulp, ITT Rayonier Angel Treated Pulp, ITT Rayonier White Jade Treated Pulp, and Coosa CR-56 Treated Pulp. Generally wood pulps can be utilized. Applicable wood pulps include chemical pulps, such as Kraft (i.e., sulfate) and sulfite pulps, as well as mechanical pulps including, for example, groundwood, thermomechanical pulp (i.e., TMP) and chemithermomechanical pulp (i.e., CTMP). Completely bleached, partially bleached and unbleached fibers are useful herein. It may frequently be desired to utilize bleached pulp for its superior brightness and consumer appeal.

Also useful in the present invention are fibers derived from recycled paper, which can contain any or all of the above categories as well as other non-fibrous materials such as fillers and adhesives used to facilitate the original paper making process.

As shown in **Figure 1**, the coform web **58** in accordance with the present invention is contacted with a relatively small amount of meltblown fibers being emitted by a meltblown extruder **60.** The meltblown fibers exiting the extruder **60** are distributed over the surface of the coform web **58** and serve to reduce lint and slough levels. The present inventors have discovered that even very small amounts of meltblown fibers distributed on the surface of the coform web significantly decrease the formation of lint and slough.

For instance, the meltblown fibers being emitted by the extruder **60** can be present on the coform web **58** in an amount less than about 8 gsm, such as less than about 6 gsm, such as less than about 4 gsm. For instance, in one embodiment, the meltblown fibers may be present on the coform web **58** in an amount from about 2 gsm to about 4 gsm.

At the above amounts, the meltblown fibers decrease lint and slough levels without substantially adversely affecting flexibility and softness. Further, the meltblown fibers may decrease the coefficient of friction of a surface of the web.

The meltblowing extruder **60** as shown In **Figure 1** generally extrudes a thermoplastic polymer resin through a plurality of small diameter capillaries of a meltblowing die as molten threads into a heated gas stream which is flowing generally in the same direction as that of the extruded threads so that the extruded threads are attenuated, i.e., drawn or extended, to reduce their diameter. Such meltblowing techniques, are discussed, for instance, in U.S. Patent No. 4,663,220 to Wisneski, et al..

The meltblown fibers exiting the extruder **60** as shown in **Figure 1** may, for instance, be In the form of continuous filaments. The filaments may have a diameter such as less than about 10 microns. For instance, the diameter of the filaments may be from about 3 microns to about 7 microns.

In general, any polymeric material capable of bonding to the coform web **58** may be extruded from the meltblown extruder **60.** Such polymers may Include, for instance, polyolefins, such as polypropylene and polyethylene. The polymeric composition may also comprise copolymers of polyolefins. In one embodiment, the polyolefin may be metallocene catalyzed, such as a metallocene catalyzed polyethylene. Such polymers are commercially available from Montell and Dow Chemical. I

As shown in **Figure 1**, the meltblown fibers exiting the extruder **60** are applied to the top surface of the coform web **58.** In an alternative embodiment, however, the meltblown fibers may be first deposited onto the forming surface **56** and the coform web **58** may be subsequently applied to the forming surface. Further, in the embodiment illustrated in **Figure 1**, only a single side of the coform web **58** is being treated with the meltblown fibers. It should be understood, however, that in other embodiments both sides of the coform web may be similarly treated with meltblown fibers. For instance, in one embodiment, the meltblown fibers may be applied to the forming surface **56** followed by the coform web **58** and later followed by an additional deposit of meltblown fibers for treating each side of the coform web.

Coform webs made according to the present invention are used as a wiping product. The coform webs may have a basis weight, for instance, from about 10 gsm to about 200 gsm. More particularly, the coform webs may have a basis weight, for example, of from about 10 gsm to about 30 gsm.

Coform webs made in accordance with the present invention are combined with other materials to form laminates.

In accordance with the present invention, the coform web is pre-saturated with a wiping solution and used as a wet wipe. The wiping solution may be any liquid which can be absorbed into the coform material to provide the desired wiping properties. For example, the wiping solution may include water, an alcohol, emollients, surfactants, fragrances, preservatives, chelating agents, pH buffers or combinations thereof. The wiping solution may also contain lotions and/or medicaments.

In one particular embodiment, the wiping solution may contain a non or low irritating silicone-based anionic sulfosuccinate. Alternatively, the wiping solution may contain a non-greasy, lubricious cleaning aid comprised of a non or low irritating long chain aliphatic anionic sulfosuccinate. In still another alternative embodiment, the cleaning solution may contain non or low irritating, hydrophilic emollient esters. The hydrophilic emollient esters may be combined with an anionic sulfosuccinate. Other optional additives that may be contained in the wiping solution include solvents, fragrances, preservatives, humectants, and other components for additional skin care benefits, such as soothing, cooling, healing, softening and the like.

In one particular embodiment of the present invention, the cleaning solution may contain a dimethicone copolyl sulfosuccinate in an amount from about 1 % to about 5% by weight, an aliphatic sulfosuccinate in an amount of from about 0.01 % to about 3% by weight and a non-ionic ester emollient in an amount of from about 0,01 % to about 2% by weight. The ester emollient can contain alkyl aliphatic or silicone derived moleties. Solvents that may be combined with the above ingredients include water, polyhydroxy compounds such as glycerin, propylene glycol, ethylene glycol, polypropylene glycol, polyethylene glycol, and the like. To the above formulation, other Ingredients such as a preservatives, fragrances, skin care agents such as Vitamin E, aloe vera, chamomile, essential oils, humectants, astringents, anti-irritants, and antioxidants may be added. The wiping solution may be applied to the coform at from about 200% to about 500% by weight of the base sheet.

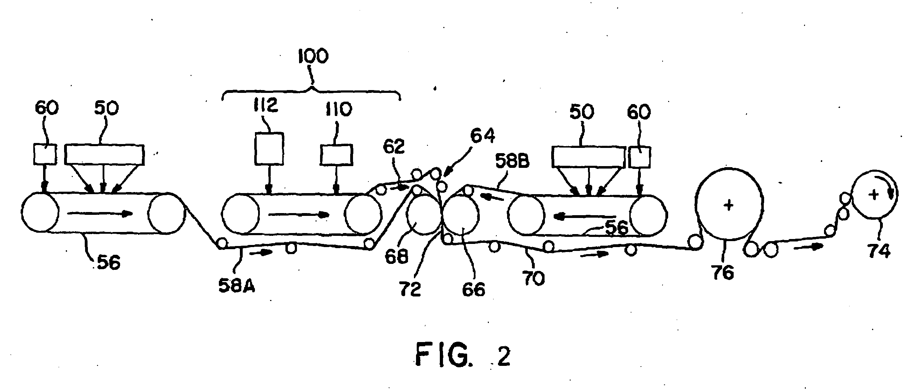

According to the present invention, conform webs made according to the description above are incorporated into a stretch-bonded laminate for forming a pre-saturated wet wipe. The stretch-bonded laminate includes a first coform web, a second coform web, and an elastic layer positioned in between the two coform webs. Each of the coform webs define an exterior surface of the laminate. Each exterior surface may be treated with meltblown fibers in accordance with the present invention for reducing lint and slough. One embodiment for forming a stretch-bonded laminate in accordance with the present invention is shown in **Figure 2**. Like reference numerals have been used to indicate similar elements.

As shown in **Figure 2**, an elastic fibrous web **62** is prepared in a web forming machine **100,** illustrated in detail in **Figure 5.** The elastic fibrous web **62** passes through a S-roll arrangement **64** before entering a horizontal calender, having a patterned calender roller **66** and an anvil roller **68.** The calender roll can have, for instance, from about 1% to about 30% embossing pin bond area, such as from about 12% to about 14%. Both the anvil and patterned rollers can be heated to provide thermal point bonding. The temperature and nip forces required to achieve adequate bonding are dependent upon the material being laminated.

A first coform web **58A** and a second coform web **58B** are prepared in accordance with the present invention as discussed in detail with respect to **Figure 1**. In particular, each coform web **58A** and **58B** is treated on an exterior surface with a light amount of meltblown fibers for reducing lint and slough. In the embodiment illustrated in **Figure 2**, as opposed to the embodiment illustrated in **Figure 1**, the meltblown extruders **60** are positioned upstream from the coform extruder banks **50.** In this manner, the meltblown fibers are first deposited onto the forming surface **56** followed by formation of the coform webs **58A** and **58B**.

The coform webs **58A** and **58B** are passed through the calender rollers **66** and **68** with the elastic layer **62**. The layers are bonded together within the calender rolls to form a stretch-bonded laminate **70.**

As shown in **Figure 2**, the elastic web **62** passes through the S-roll arrangement **64** and into a pressure nip **72** formed between the calender rollers, By controlling the peripheral linear speed of the rollers of the S-roll arrangement in relation to the peripheral linear speed of the calender rollers, the elastic fibrous web **62** is tensioned and stretched as the web is bonded to the coform webs **58A** and **58B.** The elastic web **62.** for instance, can be stretched in the range of from about 75% to about 300% of its relaxed length. For instance, the web can be stretched in the range of from about 75% to about 150% of its relaxed length, such as from about 75% to about 100% of its relaxed length.

The laminate **70** is relaxed upon release of the tensioning force by the S-roll arrangement and the calender rolls. When this occurs, the coform webs **58A** and **58B** become gathered in the resulting laminate. The stretch-bonded laminate **70** is then wound up on a winder roll **74.** Optionally, the stretch-bonded laminate **70** is activated by heat treatment in a heat activation unit **76.** Processes of making composite elastomeric materials of this type are described in, for example, U.S. Patent No. 4,720,415 to Vander Wielen, et al., U.S. Patent No. 5,385,775 to Wright, and PCT International Publication No. WO 02/053365 to Lange, et al..

The coform webs **58A** and **58B** can be joined to the elastic fibrous web **62** at least at two places by any suitable means such as, for example, thermal bonding or ultrasonic welding which softens at least portions of at least one of the materials, usually the elastic fibrous web because the elastomeric materials used for forming the elastic fibrous web **62** have a lower softening point than the components of the coform layers **58A** and **58B.** Joining can be produced by applying heat and/or pressure to the overlaid elastic fibrous web **62** and the gatherable layers **58A** and **58B** by heating these portions (or the overlaid layer) to at least the softening temperature of the material with the lowest softening temperature to form a reasonably strong and permanent bond between the resolidified softened portions of the elastic fibrous web **62** and the gatherable layers **58A** and **58B.**

The bonding roller arrangement **66, 68** includes a smooth anvil roller 68 and a patterned calender roller **66,** such as, for example, a pin embossing roller arranged with a smooth anvil roller. One or both of the smooth anvil roller and the calender roller can be heated and the pressure between these two rollers can be adjusted by well-known structures to provide the desired temperature, if any, and bonding pressure to join the gatherable layers to the elastic fibrous web. As can be appreciated, the bonding between the gatherable layers and the elastic sheet is a point bonding. Various bonding patterns can be used, depending upon the desired tactile properties of the final composite laminate material. The bonding points are preferably evenly distributed over the bonding area of the composite material.

With regard to thermal bonding, one skilled in the art will appreciate that the temperature to which the materials, or at least the bond sites thereof, are heated for heat-bonding will depend not only on the temperature of the heated roller(s) or other heat sources but on the residence time of the materials on the heated surfaces, the compositions of the material, the basis weights of the materials and their specific heats and thermal conductivities. Typically, the bonding can be conducted at a temperature of from about 40° to about 80°C. For example, the bonding can be conducted at a temperature of from about 55° to about 75° C. More preferably, the bonding can be conducted at a temperature of from about 60° to about 70° C. The typical pressure range, on the rollers, can be from about 18 to about 56.8 Kg per linear cm (KLC). For instance, the pressure range, on the rollers, can be from about 18 to about 24 Kg per linear cm (KLC).

In general, any suitable elastic layer may be incorporated Into the stretch-bonded laminate illustrated in **Figure 2.** For instance, the elastic web can be a web comprising meltblown fibers or the web can contain two or more layers of materials; where at least one layer can be a layer of elastomeric meltblown fibers and at least one layer can contain substantially parallel rows of elastomeric fibers autogenously bonded to at least a portion of the elastomeric meltblown fibers. The elastomeric fibers can have an average diameter ranging from about 40 to about 750 microns and extend along length (i.e. machine direction) of the fibrous web. The elastomeric fibers can have an average diameter in the range from about 50 to about 500 microns, for example, from about 100 to about 200 microns.

The elastic fibers extending along the length (i.e., MD) of the fibrous web increases the tensile modulus about 10% more than the tensile modulus of the fibrous web in the CD direction. For example, the tensile modulus of an elastic fibrous web can be about 20% to about 90% greater in the MD than the tensile modulus of a substantially isotropic non-woven web having about the same basis weight containing only elastomeric meltblown fibers. This increased MD tensile modulus increases the amount of retraction that can be obtained for a given basis weight of the composite elastic material.

The elastic fibrous web can contain at least about 20 percent, by weight, of elastomeric fibers. For example, the elastic fibrous web can contain from about 20 percent to about 100 percent, by weight, of the elastomeric fibers. Preferably, the elastomeric fibers can constitute from about 20 to about 60 percent, by weight, of the elastic fibrous web. More preferably, the elastomeric fibers can constitute from about 20 to about 40 percent, by weight, of the elastic fibrous web.

**Figure 3** is a schematic view of a system **100** for forming an elastic fibrous web which can be used as a component of the composite elastic material of the present invention. In forming the fibers which are used in the elastic fibrous web, pellets or chips, etc. (not shown) of an extrudable elastomeric polymer are introduced into pellet hoppers **102** and **104** of extruders **106** and **108.**

Each extruder has an extrusion screw (not shown) which is driven by a conventional drive motor (not shown). As the polymer advances through the extruder, due to rotation of the extrusion screw by the drive motor, it is progressively heated to a molten state. Heating the polymer to the molten state can be accomplished in a plurality of discrete steps with its temperature being gradually elevated as it advances through discrete heating zones of the extruder 106 toward a meltblowing die **110** and extruder **108** toward a continuous filament forming unit **112.** The meltblowing die **110** and the continuous filament forming unit **112** can be yet another heating zone where the temperature of the thermoplastic resin is maintained at an elevated level for extrusion. Heating of the various zones of the extruders **106** and **108** and the meltblowing die **110** and the continuous filament forming unit **112** can be achieved by any of a variety of conventional heating arrangements (not shown).

The elastomeric filament component of the elastic fibrous web can be formed utilizing a variety of extrusion techniques. For example, the elastic fibers can be formed utilizing one or more conventional meltblowing die units which have been modified to remove the heated gas stream (i.e., the primary air stream) which flows generally in the same direction as that of the extruded threads to attenuate the extruded threads. This modified meltblowing die unit **112** usually extends across a foraminous collecting surface **114** in a direction which is substantially transverse **to** the direction of movement of the collecting surface **114.** The modified die unit **112** includes a linear array **116** of small diameter capillaries aligned along the transverse extent of the die with the transverse extent of the die being approximately as long as the desired width of the parallel rows of elastomeric fibers which is to be produced. That is, the transverse dimension of the die is the dimension which is defined by the linear array of die capillaries. Typically, the diameter of the capillaries can be on the order of from about 0.025 cm (0.01 in) to about 0.076 cm (0.03 in). Preferably, the diameter of the capillaries can be from about 0.0368 cm (0.0145 in) to about 0.0711 cm (0.028 in). More preferably, the diameter of the capillaries can be from about 0.06 cm (0.023 in) to about 0.07 cm (0.028 in). From about 5 to about 50 such capillaries can be provided per linear inch of die face. Typically, the diameter of the capillaries can be from about 0.127 cm (0.05 in) to about 0.508 cm (0.20 in). Typically, the length of the capillaries can be about 0.287 cm (0.113 in) to about 0.356 cm (0.14 in) long. A meltblowing die can extend from about 51 cm (20 in) to about 185 or more cm (about 72 in) in length in the transverse direction. One familiar with the art would realize that the capillaries could be a shape other than circular, such as, for example, triangular, rectangular, and the like; and that the spacing or density of the capillaries can vary across the length of the die.

Since the heated gas stream (i.e., the primary air stream) which flows past the die tip is greatly reduced or absent, it is desirable to Insulate the die tip or provide heating elements to ensure that the extruded polymer remains molten and flowable while in the die tip. Polymer is extruded from the array **116** of capillaries in the modified die unit **112** to create extruded elastomeric fibers **118.**

The extruded elastomeric fibers **118** have an initial velocity as they leave the array **116** of capillaries in the modified die unit **112.** These fibers **118** are deposited upon a foraminous surface **114** which should be moving at least at the same velocity as the initial velocity of the elastic fibers **118.** This foraminous surface **114** is an endless belt conventionally driven by rollers **120.** The fibers **118** are deposited in substantially parallel alignment on the surface of the endless belt **114** which is rotating as indicated by the arrow **122 in Figure 3.** Vacuum boxes (not shown) can be used to assist in retention of the matrix on the surface of the belt **114.** The tip of the die unit **112** is as close as practical to the surface of the foraminous belt **114** upon which the continuous elastic fibers **118** are collected. For example, this forming distance can be from about 2 inches to about 10 inches. Desirably, this distance is from about 2 inches to about 8 Inches.

It may be desirable to have the foraminous surface **114** moving at a speed that is much greater than the initial velocity of the elastic fibers **118** in order to enhance the alignment of the fibers **118** into substantially parallel rows and/or elongate the fibers **118** so they achieve a desired diameter. For example, alignment of the elastomeric fibers **118** can be enhanced by having the foraminous surface **114** move at a velocity from about 2 to about 10 times greater than the initial velocity of the elastomeric fibers **118.** Even greater speed differentials can be used if desired. While different factors can affect the particular choice of velocity for the foraminous surface **114,** it will typically be from about four to about eight times faster than the initial velocity of the elastomeric fibers **118.**

Desirably, the continuous elastomeric fibers are formed at a density per inch of width of material which corresponds generally to the density of capillaries on the die face. For example, the filament density per inch of width of material may range from about 10 to about 120 such fibers per inch width of material Typically, lower densities of fibers (e.g., 10-35 fibers per inch of width) can be achieved with only one filament forming die. Higher densities (e.g., 35-120 fibers per inch of width) can be achieved with multiple banks of filament forming equipment.

The meltblown fiber component of the elastic fibrous web is formed utilizing a conventional meltblowing device **124.** Meltblowing device **124** generally extrudes a thermoplastic polymer resin through a plurality of small diameter capillaries of a meltblowing die as molten threads into a heated gas stream (the primary air stream) which is flowing generally in the same direction as that of the extruded threads so that the extruded threads are attenuated, i.e., drawn or extended, to reduce their diameter.

In the meltblown die arrangement **110,** the position of air plates which, in conjunction with a die portion define chambers and gaps, can be adjusted relative to the die portion to increase or decrease the width of the attenuating gas passageways so that the volume of attenuating gas passing through the air passageways during a given time period can be varied without varying the velocity of the attenuating gas. Generally speaking, lower attenuating gas velocities and wider air passageway gaps are generally preferred if substantially continuous meltblown fibers or microfibers are to be produced.

The two streams of attenuating gas converge to form a stream of gas which entrains and attenuates the molten threads, as they exit the orifices, into fibers depending upon the degree of attenuation, microfibers, of a small diameter which is usually less than the diameter of the orifices. The gas-borne fibers or microfibers **126** are blown, by the action of the attenuating gas, onto a collecting arrangement which, in the embodiment illustrated in **Figure 3**, is the foraminous endless belt **114** which carries the elastomeric filament in substantially parallel alignment The fibers or microfibers **126** are collected as a coherent matrix of fibers on the surface of the elastomeric fibers **118** and foraminous endless belt **114,** which is rotating clockwise as indicated by the arrow **122** in **Figure 3**. If desired, the meltblown fibers or microfibers **126** can be collected on the foraminous endless belt **114** at numerous impingement angles. Vacuum boxes (not shown) can be used to assist in retention of the matrix on the surface of the belt **114**. Typically the tip **128** of the die **110** is from about 6 inches to about 14 inches from the surface of the foraminous belt **114** upon which the fibers are collected. The entangled fibers or microfibers **124** autogenously bond to at least a portion of the elastic continuous fibers **118** because the fibers or microfibers **124** are still somewhat tacky or molten while they are deposited on the elastic continuous fibers **118,** thereby forming the elastic fibrous web **130.** The fibers are quenched by allowing them to cool to a temperature below about 38° C.

As discussed above, the elastomeric fibers and elastomeric meltblown fibers can be deposited upon a moving foraminous surface. In one embodiment of the invention, meltblown fibers can be formed directly on top of the extruded elastomeric fibers. This is achieved by passing the fibers and the foraminous surface under equipment which produces meltblown fibers. Alternatively, a layer of elastomeric meltblown fibers can be deposited on a foraminous surface and substantially parallel rows of elastomeric fibers can be formed directly upon the elastomeric meltblown fibers. Various combinations of filament forming and fiber forming equipment can be set up to produce different types of elastic fibrous webs. For example, the elastic fibrous web may contain alternating layers of elastomeric fibers and elastomeric meltblown fibers. Several dies for forming meltblown fibers or creating elastomeric fibers may also be arranged in series to provide superposed layers of fibers.

The elastomeric meltblown fibers and elastomeric fibers can be made from any material that can be manufactured into such fibers such as natural polymers or synthetic polymers. Generally, any suitable elastomeric fiber forming resins or blends containing the same can be utilized for the elastomeric meltblown fibers and any suitable elastomeric filament forming resins or blends containing the same can be utilized for the elastomeric fibers. The fibers can be formed from the same or different elastomeric resin.

For example, the elastomeric meltblown fibers and/or the elastomeric fibers can be made from block copolymers having the general formula A-B-A' where A and A' are each a thermoplastic polymer endblock which can contain a styrenic moiety such as a poly (vinyl arene) and where B is an elastomeric polymer midblock such as a conjugated diene or a lower alkene polymer. The block copolymers can be, for example, (polystyrene/poly(ethylene-butylene)/polystyrene) block copolymers available from the Shell Chemical Company under the trademark KRATON R™G. One such block copolymer can be, for example, KRATON R™G-1657.

Other exemplary elastomeric materials which can be used include polyurethane elastomeric materials such as, for example, those available under the trademark ESTANE from B.F. Goodrich & Co., polyamide elastomeric materials such as, for example, those available under the trademark PEBAX from the Rilsan Company, and polyester elastomeric materials such as, for example, those available under the trade designation Hytrel from E.I. DuPont De Nemours & Company. Formation of elastomeric meltblown fibers from polyester elastic materials is disclosed in, for example, U.S. Patent No. 4,741,949 to Morman, et al.

Useful elastomeric polymers also include, for example, elastic copolymers of ethylene and at least one vinyl monomer such as, for example, vinyl acetates, unsaturated aliphatic monocarboxylic acids, and esters of such monocarboxylic acids. The elastic copolymers and formation of elastomeric meltblown fibers from those elastic copolymers are disclosed in, for example, U.S. Patent No. 4,803,117 to Daponte. Also, suitable elastomeric polymers are those prepared using metallocene catalysts such as those disclosed in International Application WO 00/48834 to Smith, et al.

Processing aids can be added to the elastomeric polymer. For example, a polyolefin can be blended with the elastomeric polymer (e.g., the A-B-A elastomeric block copolymer) to improve the processability of the composition. The polyolefin must be one which, when so blended and subjected to an appropriate combination elevated pressure and elevated temperature conditions, extrudable, in blended form, with the elastomeric polymer. Useful blending polyolefin materials include, for example, polyethylene, polypropylene and polybutylene, including ethylene copolymers, propylene copolymers and butylene copolymers. A particularly useful polyethylene can be obtained from the U.S.I. Chemical Company under the trade designation Betrothing NA 601 (also referred to herein as PE NA 601 or polyethylene NA 601). Two or more of the polyolefins can be utilized. Extrudable blends of elastomeric polymers and polyolefins are disclosed in, for example, previously referenced U.S. Patent No. 4,663,220 to Wisneski, et al.

The elastomeric meltblown fibers and/or the elastomeric fibers can have some tackiness adhesiveness to enhance autogenous bonding. For example, the elastomeric polymer itself can be tacky when formed into fibers or, optionally, a compatible tackifying resin can be added to the extrudable elastomeric compositions described above to provide tackified elastomeric fibers and/or fibers that autogenously bond. In regard to the tackifying resins and tackified extrudable elastomeric compositions, note the resins and compositions as disclosed in U.S. Patent No. 4,787,699, to Moulin.

Any tackified resin can be used which is compatible with the elastomeric polymer and can withstand the high processing (e.g., extrusion) temperatures. If the elastomeric polymer (i.e., A-B-A elastomeric block copolymer) is blended with processing aids such as, for example, polyolefins or extending oils, the tackifier resin should also be compatible with those processing aids. Generally, hydrogenated hydrocarbon resins are preferred tackifying resins, because of their better temperature stability. Composite elastic material REGALREZ™ and ARKON™ series tackifiers are examples of hydrogenated hydrocarbon resins. ZONATAK™ 501 Lite is an example of a terpene hydocarbon. REGALREZ™ hydrocarbon resins are available from Hercules Incorporated. ARKON™ series resins are available from Arakawa Chemical (U.S.A.) Inc. The present invention is not limited to use of these tackifying resins, and other tackifying resins which are compatible with the other components of the composition and can withstand the high processing temperatures, can also be used.

Typically, the blend used to form the elastomeric fibers include, for example, from about 40 to about 95 percent by weight elastomeric polymer, from about 5 to about 40 percent polyolefin and from about 5 to about 40 percent resin tackified. For example, a particularly useful composition included, by weight, about 61 to about 65 percent KRATON™ G-1657, about 17 to about 23 percent polyethylene polymer, and about 15 to about 20 percent Composite elastic material REGALREZ™ 1126. The preferred polymers are metallocene catalyzed polyethylene polymers, such as, for example Affinity® polymers, available from Dow® Chemical Company as Affinity XUS59400.03L.

The elastomeric meltblown fiber component of the present invention can be a mixture of elastic and non-elastic fibers or particulates. For example, such mixture, is disclosed in U.S. Patent No. 4,209,563 to Sisson, where elastomeric and non-elastomerlc fibers are commingled to form a single coherent web of randomly dispersed fibers. Another example of such an elastic composite web could be made by a technique disclosed in previously cited U.S. Patent No. 4,741,949 to Morman et al. This patent discloses an elastic non-woven material which includes a mixture of meltblown thermoplastic, fibers and other materials. The fibers and other material are combined in the gas stream in which the meltblown fibers are borne so that an intimate entangled commingling of meltblown fibers and other material, e.g., wood pulp, staple fibers or particulates such as, for example, activated charcoal, clays, starches, or hydrocolloid (hydrogel) particulates commonly referred to as super absorbents occurs prior to collection of the fibers upon a collecting device to form a coherent web of randomly dispersed fibers.

Once the stretch-bonded laminate is formed, such as according to the process shown in **Figure 2**, the material is cut into a desired shape and impregnated with a cleaning solution for forming a wet wipe. For instance, each wet wipe may generally be rectangular in shape, and may have any suitable unfolded width and length. For example, the wet wipe may have an unfolded length of from about 2.0 to about 80.0 centimeters and desirable from about 10.0 to about 25.0 centimeters and an unfolded width of from about 2.0 to about 80.0 centimeters and desirably from about 10.0 to about 25.0 centimeters. Preferably, each individual wet wipe is arranged in a folded configuration and stacked one on top of the other to provide a stack of wet wipes or interfolded in a configuration suitable for pop-up dispensing. Such folded configurations are well known to those skilled in the art and include c-folded, z-folded, quarter-folded configurations and the like. The stack of folded wet wipes can be placed in the interior of a container, such as a plastic tub, to provide a package of wet wipes for eventual sale to the consumer. Alternatively, the wet wipes may include a continuous strip of material which has perforations between each wipe and which can be arranged in a stack or wound into a roll for dispensing.

The present invention may be better understood with respect to the following examples.

## EXAMPLE NO. 1

To illustrate the properties of the product made in accordance with the present invention, tests were conducted on several samples of wet wipe materials in order to investigate the properties of each. Included in this example are samples of 3 general types of materials. The first was a control group including a three layered laminated article with two coform outer layers and an elastomeric inner layer as described in the current application. The second, also described in the above application, was a similar product to the first control group, but with an added polypropylene meltblown layer of varying thickness on the exposed surfaces of the outer coform layers. The third type of sample was a high pulp content nonwoven composite fabric, available from the Kimberly Clark Corp, under the registered trademark Hydroknit (HK). The samples with polypropylene meltblown exposed layer had veneer thicknesses of 4 gsm, 6 gsm, 8 gum, and 10 gsm , resulting in a total of 6 sample groups including the control and Hydroknit samples. The samples have been abbreviated: Control, HK, 4 gsm Veneer, 6 gsm Veneer. 8gsm Veneer, and 10 gsm Veneer.

The Control and Veneer samples were produced as described in the above application and as shown in **Figure 2**. However, in the case of the control sample, the meltblown bank 60 was not used. The target basis weight for each of the outer layers (conform + veneer) was 26 gsm, which resulted In an overall laminate basis weight of approximately 87 gsm (see Table I for specific values). In order to achieve a constant outer layer basis weight, the flow rates for the meltblown banks were altered such that for increasing veneer basis weights, the coform bank flow rates were decreased and the veneer bank flow rates were increased.

The Hydroknit sample was produced by the method described in U.S. Pat. No. 5,284,703 to Everhart, et al. entitled "High Pulp Content Non-Woven Composite Fabric". The composite fabric contains more than about 70 percent, by weight, pulp fibers which are hydraulically entangled into a continuous filament substrate. The process basically comprises wet laid pulp being added to a spunbound filament.

For each sample a roll was prepared and then slit into 8.5" x 8.5" sheets, which were then folded according to a modified N-fold prior to wetting. The prepared sheets were then wetted with a wetting solution, which was applied to the wipes using a stainless steel pipe with holes from which the solution was allowed to fall onto the wipes, resulting in a product similar to that available to consumers. The wipes were wetted with the solution to a 250% add-on level and placed in sealed ZIP-LOCK bags. The wet wipes were then subjected to a series of standardized tests. All tests were conducted with constant laboratory conditions of 23±2°C and 50±5% humidity unless otherwise stated. Table I below shows the most relevant physical data for each of the 6 samples, including: basis weight (gsm), bulk (mm), absorption capacity (g/g), coefficient of friction (COF) in the machine direction (MD), cup crush energy (g*mm), tensile strength in the machine direction (MD) and tensile strength in the cross-machine direction (CD).

The bulk of the samples is a measure of thickness. The bulk is measured at 0.05 psi of pressure with a Starret-type bulk tester, in units of millimeters (mm). The tester uses a 7.6 cm (3 in.) diameter platen, and care must be taken to insure the platen does not fall on a fold or wrinkle that has resulted from packaging and/or folding.

The absorption capacity of paper products (either their water or oil absorbent capacities) may be determined according to the following procedure. A pan large enough to hold water to a depth of at least 2 inches (5.08 cm) is filled with distilled water (or oil). A balance, such as the OHAUS GT480 balance, is utilized in addition to a stopwatch. A cutting device, such as that sold under the trade designation TMI DGD by Testing Machines, Inc., of Amityville, N.Y., and a die with dimensions of 4 inches by 4 inches (±0.01 inches) (10.16 cm by 10.16 cm± 0.25 cm) are also utilized. Specimens of the die size are cut and weighed dry to the nearest 0.01 gram. The stopwatch is started when the specimen is placed in the pan of water (or oil) and soaked for 3 minutes ±5 seconds. At the end of the specified time, the specimen is removed by forceps and attached to a hanging clamp to hang in a "diamond" shaped position to ensure the proper flow of fluid from the specimen. In addition, the specimen is hung in a chamber having 100 percent relative humidity for 3 minutes ±5 seconds. The specimen is then allowed to fall into the weighing dish upon releasing the clamp. The weight is then recorded to the nearest 0.01 gram. The absorbent or absorptive capacity of each specimen is then calculated as follows:

$$\text{Absorbent Capacity (g)} = \text{Wet weight (g)} - \text{Dry weight (g)}$$

This gives an absorption capacity in grams for the sample which is often reported per weight of sample, giving a specific absorption capacity with units of grams absorbed per grams of sample, as reported in Table I.

The coefficient of friction can be measured with known devices, which drag a probe over the surface of a paper sample at a constant rate. The probe is modified to be a circular 2-centimeter diameter 40-60 micron glass frit, lying flat, applying a 12.5 g normal force to the sample, and it is advanced over the tissue at a rate of 1 mm/sec. The probe is advanced 5 cm in a first direction, providing data for a "forward" scan, and then is reversed to travel back to the beginning point at the same speed, providing data for the "reverse" scan. The coefficient of friction can be calculated by dividing the frictional force by the normal force measured during the scan (neglecting the initial static resistance). The frictional force is the lateral force on the probe during the scanning, an output of the instrument. After a first test comprising a forward and reverse scan, the sample is rotated 180 degrees and repositioned for a second test with another forward and reverse pair of scans along a new path, such that the forward scan of the second test is in the same direction as the reverse scan in the first test. The coefficient of friction for the forward scan of the second test and the reverse scan in the first test are averaged to give the coefficient of friction in a first direction, and the coefficient of friction for the reverse scan of the second test and the forward scan in the first test are averaged to give the coefficient of friction in a second direction opposite to the first direction. This process is repeated for 10 samples to yield averaged coefficients of frictions for the two directions.

The softness of a nonwoven fabric may be measured according to the "cup crush" test. The cup crush test evaluates fabric stiffness by measuring the peak load (also called the "cup crush load" or just "cup crush") required for a 4.5 cm diameter hemispherically shaped foot to crush a 23 cm by 23 cm piece of fabric shaped into approximately 6.5 cm diameter by 6.5 cm tall inverted cup while the cup shaped fabric is surrounded by an approximately 6.5 cm diameter cylinder to maintain a uniform deformation of the cup shaped fabric. An average of 10 readings is used. The foot and the cup are aligned to avoid contact between the cup walls and the foot which could affect the readings. The peak load is measured while the foot is descending at a rate of about 0.25 inches per second (380 mm per minute) and is measured in grams. The cup crush test also yields a value for the total energy required to crush a sample (the cup crush energy) which is the energy from the start of the test to the peak load point, i.e. the area under the curve formed by the load in grams on the one axis and the distance the foot travels in millimeters on the other. Cup crush energy is therefore reported in g*mm. Lower cup crush values indicate a softer laminate. A suitable device for measuring cup crush is a model FTD-G-500 load cell (500 gram range) available from the Schaevitz Company of Pennsauken, NJ.

The peak load tensile test is a measure of breaking strength and elongation or strain of a fabric when subjected to a unidirectional stress. This test is known in the art and is similar to ASTM-1117-80 § 7, which uses a 12-inch per minute strain rate. The results are expressed in grams to break and percent stretch before breakage. Higher numbers indicate a stronger, more stretchable fabric. The term "load" means the maximum load or force, expressed in units of weight, required to break or rupture the specimen in a tensile test. Values for tensile strength are obtained using a specified width of fabric, clamp width and a constant rate of extension. The test is conducted using a wet product as would be representative of consumer use. Fabric testing can be conducted in both the machine direction and cross-machine direction, which can be determined by one familiar with non-woven materials by the orientation of the fibers. It is important that the samples be either parallel or perpendicular to the machine direction to insure accuracy. The test is conducted using a 4 inch wide clamp with one smooth face and one 0.25 inch round horizontal rod comprising each clamp mechanism. The specimen is clamped in, for example, an Instron Model TM, available from the Instron Corporation of Canton, MA, or a Thwing Albert Model INTELLECT II available from the Thwing Albert Instrument Co. of Philadelphia, PA, which have 3-inch long parallel clamps. This closely simulates fabric stress conditions in actual use.

| Table I: Physical Data | | | | | | | |
|---|---|---|---|---|---|---|---|
| Sample | Basis Weight (gsm) | Bulk (mm) | Absorption Capacity (g/g) | COF MD (sheet/sheet) | Cup Crush (g*mm) | MD Tensile (lb/in) | CD Tensile (lb/in) |
| Control | 86.6 | 1.22 | 6.61 | 1.62 | 1110 | 2.10 | 0.95 |
| 4 gsm Veneer | 86.7 | 1.39 | 6.97 | 1.14 | 1190 | 2.53 | 1.21 |
| 6 gsm Veneer * | 82.7* | 1.10* | 6.76* | 1.07* | 1330* | 2.53* | 1.34* |
| 8 gsm Veneer | 86.5 | 1.32 | 6.91 | 1.09 | 1670 | 2.71 | 1.54 |
| 10 gsm Veneer | 87.5 | 1.44 | 6.50 | 1.13 | 1680 | 3.19 | 1.73 |

(continued)

| Table I: Physical Data | | | | | | | |
|---|---|---|---|---|---|---|---|
| Sample | Basis Weight (gsm) | Bulk (mm) | Absorption Capacity (g/g) | COF MD (sheet/ sheet) | Cup Crush (g*mm) | MD Tensile (lb/in) | CD Tensile (lb/in) |
| HK | 66.4 | 0.48 | 5.83 | 1.83 | 1040 | 3.55 | 2.16 |
| * Process problems experienced during production of 6 gsm Veneer wipes resulted in erroneous data for that sample, as indicated most obviously by the low basis weight. This production problem should be considered when evaluating any data on this sample. | | | | | | | |

One advantage provided by the meltblown surface layer is a reduction in lint production. In order to quantify this advantage, a wet wipe lint test was conducted. Once again, 8.5" x 8.5" wet wipes were used. The test was conducted by placing one wipe from each of the sample groups into a 5L or larger container containing 2L of distilled water. The wipe was then swirled in a clockwise direction for 30 seconds. A sample of the resulting solution was then poured into a smaller jar. That solution was tested for particles of varying sizes using a HIAC/ROYCO Automatic Bottle Sampler (ABS-2) and a HIAC/ROYCO Model 8000A/8000S Particle Counter, both available from Pacific Scientific Instruments of Grant Pass, OR. The number of lint particles from each sample was counted and separated by size. The results of the test are shown in Table II below.

| Table II : Wet Wipe Lint Test Data | | | | | | |
|---|---|---|---|---|---|---|
| Particle Size (microns) | Control | 4 gsm Veneer | 6 gsm Veneer* | 8 gsm Veneer | 10 gsm Veneer | HK |
| 5 | 317000 | 185000 | 198000 | 183000 | 168000 | 87200 |
| 10 | 163000 | 69300 | 74500 | 68700 | 61500 | 12600 |
| 25 | 14300 | 4950 | 5100 | 4640 | 3860 | 1800 |
| 50 | 149 | 61 | 81 | 74 | 56 | 60 |
| 60 | 151 | 54 | 80 | 64 | 57 | 44 |
| 100 | 24 | 7 | 10 | 10 | 6 | 8 |
| 500+ | 0 | 0 | 0 | 0 | 0 | 0 |
| *See note below Table I | | | | | | |

As shown above, the meltblown veneer of the present invention significantly reduced lint levels when compared to the control. Further, the 4 gsm meltblown veneer produced similar results when compared to the 10 gsm meltblown veneer.

When conducting lint tests as shown above, particle sizes of 50 microns or greater are perhaps of more concern since these particles are visible to the user. As shown, meltblown veneers made according to the present invention may reduce lint levels by greater than about 30%, such as greater than about 40%, such as greater than about 50%, such as greater than about 60%, and, in one embodiment, may reduce lint levels by greater than about 70%.

## EXAMPLE NO. 2

To demonstrate the utility of webs treated according to the present invention, a pilot meltblown line was operated to provide a light meltblown coating of Findley H-1296 adhesive made by Bostik Findley, Inc. (Middleton, Massachusetts), which is believed to comprise ethylene vinyl acetate. The trials were conducted on a J&M meltblown line made by J&M Laboratories, Inc. (Dawsonville, Georgia). The meltblown was applied onto webs of uncalendered, uncreped through-air dried (UCTAD) tissue basesheets, made generally according to the teachings of U.S. Patent No. 5,672,248, issued to Wendt, et al. on Sept. 30, 1997, and U.S. Patent No. 5,607,551, issued to Farrington et al. on March 4, 1997.

A first UCTAD tissue basesheet comprised a three-layered web formed using a stratified headbox. The two outer layers each had a target basis weight of 8 grams per square meter (gsm) of 100% bleached kraft Alabama hardwood with debonder added at a level of 5.1 kg per metric tonne of fiber. The debonder was PROSOFT® TQ1003 debonder, an imidazoline debonder (more specifically, an oleylimidazolinium debonder) manufactured by Hercules Inc., (Wilmington, Delaware) which inhibits hydrogen bonding, resulting in a weaker sheet. The inner layer of the basesheet contained lightly refined 100% LL19 bleached kraft northern softwood fibers from Kimberly-Clark Corp. (Houston, Texas) with PAREZ® 631-NC strength additive, made by Bayer AG (Leverkusen, Germany), added at a level of 4 kg per metric tonne of fibers.

The UCTAD basesheet was formed using 25% rush transfer and dried on a textured through-drying fabric to impart a three-dimensional pattern substantially the same as the pattern on commercial KLEENEX® COTTONELLE® toilet paper. The resulting basesheet had a total basis weight of 30 gsm and a geometric mean tensile strength of 750 grams per 3 inches. However, unlike the related commercial toilet paper, the basesheet used in this example had a composition designed to provide high slough and lint problems, particularly due to the composition of the outer layers. Prior to treatment with the meltblown, the air-side of the basesheet (the side that was not against the through dryer surface during drying) was observed to release dust or lint (typically hardwood fibers) when rubbed. Since the air side of a through-dried web generally experiences less mechanical compaction during drying than does the side against the through dryer surface, the air-side can be less bonded and thus more likely to slough or release lint under frictional forces.

The dry UCTAD web with the air-side up was then placed on a moving carrier wire in the meltblown line which conveyed the web at a speed of 81 feet per minute to pass beneath a meltblowing die 1.5 inches above the web with a spray width of 12 inches. The hotmelt tank was at 330°F, the die tip at 325°F, and the air temperature was 375°F. The hotmelt pump operated at 15 grams per minute. Meltblown fibers from the die tip were deposited on the tissue web, resulting in a light meltblown layer well attached to the web and a basis weight of about 2 grams per square meter on one side of the tissue.

After treatment, the low-basis weight meltblown fibers were not visible to the unaided eye, but the treated side of the web that previously was subject to dust or lint formation was much more lint resistant. The web remained absorbent and had a soft, pleasant tactile feel with higher surface friction than the untreated side due to the presence of the meltblown fibers.

Additional trials were conducted at about 160 feet per minute, yielding a meltblown layer with a basis weight of about 1.1 gsm

Trials were also conducted with a second UCTAD basesheet substantially the same as the first UNCTAD basesheet, except that the outer layers contained 50% bleached kraft eucalyptus and 50% bleached kraft Alabama hardwood, still with outer layer basis weights of 8 gsm and still having 5.1 kg/tonne of the debonder present.

With the second UCTAD basesheet, meltblown trials were conducted at speed at 81 feet per minute, 161 feet per minute, and 320 feet per minute, yielding meltblown layers on the air-side of the basesheet with basis weights of, respectively, about 2 gsm, about 1 gsm, and about 0.5 gsm.

In another trial, the basesheet was a 40 gsm basesheet of 100% northern softwood bleached chemithermomechanical pulp (BCTMP), made substantially according to Example 1 of U.S. Patent No. 6,436,234, issued Aug. 20, 2002 to Chen, et al. The meltblown line was operated at 120 feet per minute to apply about 1.5 gsm of meltblown to a first side of the basesheet. The treated basesheet was placed on a roll, and then brought to the front of the machine again, where it was unwound with the untreated side up to treat the second side of the web. Thus, meltblown was applied to both sides of the basesheet.

## Claims

1. A wet wipe comprising:

    a stretch-bonded laminate (70) comprising a first coform web (58A), a second coform web (58B) and an elastic layer (62) located in between the first coform web (58A) and the second coform web (58B), the first coform web (58A) defining a first exterior side of the stretch-bonded laminate and the second coform web (58B) defining a second exterior side of the stretch-bonded laminate;
    meltblown fibers applied to the first exterior side and to the second exterior side of the stretch-bonded laminate (70), the meltblown fibers being distributed over the surfaces of the stretch-bonded laminate (70), the meltblown fibers being present on each side of the stretch-bonded laminate (70) in an amount less than about 8 gsm; and
    a wiping solution impregnated into the stretch-bonded laminate (70).

2. A wet wipe as defined in claim 1, wherein the meltblown fibers are present in an amount less than about 6 gsm.

3. A wet wipe as defined in claim 1, wherein the meltblown fibers are present in an amount less than about 4 gsm.

4. A wet wipe as defined in claim 1, wherein the meltblown fibers are present in an amount less than about 2 gsm.

**5.** A wet wipe as defined in claim 1, wherein the meltblown fibers are present in an amount less than about 1 gsm.

**6.** A wet wipe as defined in any one of the preceding claims, wherein the meltblown fibers are made from a material selected from the group consisting of styrene-butadiene copolymers, polyvinyl acetate homopolymers, ethylene vinyl acetate copolymers, ethylene vinyl alcohol, vinyl acetate acrylic copolymers, ethylene vinyl chloride copolymers, ethylene vinyl chloride-vinyl acetate terpolymers, acrylic polyvinyl chloride polymers, acrylic polymers, waxes, and mixtures thereof.

**7.** A wet wipe as defined in any one of the preceding claims, wherein the meltblown fibers comprise continuous filaments having a diameter of less than about 10 microns.

**8.** A wet wipe as defined in any one of claims 1-6, wherein the meltblown fibers comprise continuous filaments having a diameter of less than about 5 microns.

**9.** A wet wipe as defined in any one of the preceding claims, wherein the coform web comprises polyolefin fibers and pulp fibers and wherein the meltblown fibers comprise polyolefin fibers.

**10.** A wet wipe as defined in any one of the preceding claims having a cup crush of less than about 120 g/cm.

**11.** A wet wipe as defined in any one of the preceding claims, wherein the meltblown fibers decrease lint levels for particles greater than 50 microns by at least about 30%.

**12.** A wet wipe as defined in any one of claims 1-10, wherein the meltblown fibers decrease lint levels for particles greater than 50 microns by at least about 40%.

**13.** A wet wipe as defined in any one of claims 1-10, wherein the meltblown fibers decrease lint levels for particles greater than 50 microns by at least about 50%.

**Patentansprüche**

**1.** Ein Feuchttuch, welches umfasst:

ein streckgebundenes Laminat (70) welches eine erste Coform-Bahn (58A), eine zweite Coform-Bahn (58B) und eine elastische Schicht (62) umfasst, welche sich zwischen der ersten Coform-Bahn (58A) und der zweiten Coform-Bahn (58B) befindet, wobei die erste Coform-Bahn (58A) eine erste äußere Seite des streckgebundenen Laminats definiert und wobei die zweite Coform-Bahn (58B) eine zweite äußere Seite des streckgebundenen Laminats definiert;
schmelzgeblasene Fasern, welche auf die erste äußere Seite und auf die zweite äußere Seite des streckgebundenen Laminats (70) aufgebracht sind, wobei die schmelzgeblasenen Fasern über die Oberflächen des streckgebundenen Laminats (70) verteilt sind, wobei die schmelzgeblasenen Fasern auf jeder Seite des streckgebundenen Laminats (70) in einer Menge von weniger als ungefähr 8 g/qm vorhanden sind; und
eine Wischlösung, welche in das streckgebundene Laminat imprägniert ist.

**2.** Ein Feuchttuch gemäß Anspruch 1, wobei die schmelzgeblasenen Fasern in einer Menge von weniger als ungefähr 6 g/qm vorhanden sind.

**3.** Ein Feuchttuch gemäß Anspruch 1, wobei die schmelzgeblasenen Fasern in einer Menge von weniger als ungefähr 4 g/qm vorhanden sind.

**4.** Ein Feuchttuch gemäß Anspruch 1, wobei die schmelzgeblasenen Fasern in einer Menge von weniger als ungefähr 2 g/qm vorhanden sind.

**5.** Ein Feuchttuch gemäß Anspruch 1, wobei die schmelzgeblasenen Fasern in einer Menge von weniger als ungefähr 1 g/qm vorhanden sind.

**6.** Ein Feuchttuch gemäß einem der vorherigen Ansprüche, wobei die schmelzgeblasenen Fasern aus Material hergestellt sind ausgewählt aus der Gruppe bestehend aus Styren-Butadien-Copolymeren, Polyvinylacetat-Homopolymeren, Ethylen-Vinylacetat-Copolymeren, Ethylen-Vinyl-Alkohol, Vinylacetat-Acryl-Copolymeren, Ethylen-Vinylchlorid-Copolymeren, Ethylen-Vinylchlorid-Vinylacetat-Terpolymeren, Acryl-Polyvinylchlorid-Polymeren, Acryl-Polymeren, Wachsen und Mischungen davon.

**7.** Ein Feuchttuch gemäß einem der vorherigen Ansprüche, wobei die schmelzgeblasenen Fasern kontinuierliche Filamente mit einem Durchmesser von weniger als ungefähr 10 Mikrometern umfassen.

**8.** Ein Feuchttuch gemäß einem der Ansprüche 1-6, wobei die schmelzgeblasenen Fasern kontinuierliche Filamente mit einem Durchmesser von weniger als ungefähr 5 Mikrometern umfassen.

**9.** Ein Feuchttuch gemäß einem der vorherigen Ansprüche, wobei die Coform-Bahn Polyolefinfasern und Pulpefasern umfasst, und wobei die schmelzgeblasenen Fasern Polyolefinfasern umfassen.

**10.** Ein Feuchttuch gemäß einem der vorherigen Ansprüche, welches einen Cup-Crush von weniger als ungefähr 120 g/cm aufweist.

**11.** Ein Feuchttuch gemäß einem der vorherigen Ansprüche, wobei die schmelzgeblasenen Fasern Flusenlevel (lint levels) für Teilchen größer als 50 Mikrometer um mindestens 30% verringern.

**12.** Ein Feuchttuch gemäß einem der Ansprüche 1-10, wobei die schmelzgeblasenen Fasern Flusenlevel (lint levels) für Teilchen größer als 50 Mikrometer um mindestens 40% verringern.

**13.** Ein Feuchttuch gemäß einem der Ansprüche 1-10, wobei die schmelzgeblasenen Fasern Flusenlevel (lint levels) für Teilchen größer als 50 Mikrometer um mindestens 50% verringern.

**Revendications**

**1.** Lingette humide comprenant :

un stratifié étiré sous striction (70) comprenant un premier voile coformé (58A), un second voile coformé (58B) et une couche élastique (62) située entre le premier voile coformé (58A) et le second voile coformé (58B), le premier voile coformé (58A) définissant une première face extérieure du stratifié étiré sous striction et le second voile coformé (58B) définissant une seconde face extérieure du stratifié étiré sous striction ;
des fibres obtenues par extrusion-soufflage appliquées à la première face extérieure et à la seconde face extérieure du stratifié étiré sous striction (70), les fibres obtenues par extrusion-soufflage étant distribuées sur les surfaces du stratifié étiré sous striction (70), les fibres obtenues par extrusion-soufflage étant présentes sur chaque face du stratifié étiré sous striction (70) en une quantité inférieure à environ 8 g/m$^2$ ; et
une solution d'essuyage imprégnant le stratifié étiré sous striction (70).

**2.** Lingette humide telle que définie dans la revendication 1, dans laquelle les fibres obtenues par extrusion-soufflage sont présentes en une quantité inférieure à environ 6 g/m$^2$.

**3.** Lingette humide telle que définie dans la revendication 1, dans laquelle les fibres obtenues par extrusion-soufflage sont présentes en une quantité inférieure à environ 4 g/m$^2$.

**4.** Lingette humide telle que définie dans la revendication 1, dans laquelle les fibres obtenues par extrusion-soufflage sont présentes en une quantité inférieure à environ 2 g/m$^2$.

**5.** Lingette humide telle que définie dans la revendication 1, dans laquelle les fibres obtenues par extrusion-soufflage sont présentes en une quantité inférieure à environ 1 g/m$^2$.

**6.** Lingette humide telle que définie dans l'une quelconque des revendications précédentes, dans laquelle les fibres obtenues par extrusion-soufflage sont faites d'un matériau sélectionné dans le groupe consistant en les copolymères styrène-butadiène, les homopolymères poly(acétate de vinyle), les copolymères éthylène-acétate de vinyle, l'éthylène-alcool vinylique, les copolymères acétate de vinyle-acrylique, les copolymères éthylène-chlorure de vinyle, les copolymères éthylène-chlorure de vinyle-acétate de vinyle, les polymères acrylique-poly(chlorure de vinyle), les polymères acryliques, les cires et leurs mélanges.

**7.** Lingette humide telle que définie dans l'une quelconque des revendications précédentes, dans laquelle les fibres obtenues par extrusion-soufflage comprennent des filaments continus ayant un diamètre inférieur à environ 10 microns.

**8.** Lingette humide telle que définie dans l'une quelconque des revendications 1-6, dans laquelle les fibres obtenues par extrusion-soufflage comprennent des filaments continus ayant un diamètre inférieur à environ 5 microns.

**9.** Lingette humide telle que définie dans l'une quelconque des revendications précédentes, dans laquelle le voile coformé comprend des fibres de polyoléfine et des fibres de pâte et dans laquelle les fibres obtenues par extrusion-soufflage comprennent des fibres de polyoléfine.

**10.** Lingette humide telle que définie dans l'une quelconque des revendications précédentes ayant une valeur au test d'écrasement de la coupelle inférieure à environ 120 g/cm.

**11.** Lingette humide telle que définie dans l'une quelconque des revendications précédentes, dans laquelle les fibres obtenues par extrusion-soufflage abaissent d'au moins 30 % le niveau de peluchage pour des particules supérieures à 50 microns.

**12.** Lingette humide telle que définie dans l'une quelconque des revendications 1-10, dans laquelle les fibres obtenues par extrusion-soufflage abaissent d'au moins 40 % le niveau de peluchage pour des particules supérieures à 50 microns.

**13.** Lingette humide telle que définie dans l'une quelconque des revendications 1-10, dans laquelle les fibres obtenues par extrusion-soufflage abaissent d'au moins 50 % le niveau de peluchage pour des particules supérieures à 50 microns.

FIG. 1

FIG. 2

FIG. 3

EP 1 704 273 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9004060 A
- EP 0205242 A
- US 3849241 A, Butin
- US 4340563 A, Appel
- US 3692618 A, Dorschner
- US 4100324 A, Anderson
- US 5508102 A, Georger
- EP 0590307 A
- US 5284703 A, Everhart
- US 5350624 A, Georger
- US 5385775 A, Wright
- US 4720415 A, Vander Wielen
- US 4663220 A, Wisneski
- WO 02053365 A, Lange
- US 4741949 A, Morman
- US 4803117 A, Daponte
- WO 0048834 A, Smith
- US 4787699 A, Moulin
- US 4209563 A, Sisson
- US 5672248 A, Wendt
- US 5607551 A, Farrington
- US 6436234 B, Chen